# EUROPEAN PATENT APPLICATION

(11) **EP 3 795 264 A1**
(43) Date of publication of application: **24.03.2021**
(21) Application number: 19198378.2
(22) Date of filing: 19.09.2019
(51) Int. Cl.: B08B 17/02, B63B 59/04, B01J 19/12

(54) **METHOD AND SYSTEM FOR PROTECTING A SURFACE AGAINST BIOFOULING**

(71) Applicant: Koninklijke Philips N.V., 5656 AG Eindhoven (NL)
(72) Inventor: VAN UDEN, Martijn Gerarda Lambertus Justinus, 5656 AE Eindhoven (NL); LEIJSSEN, Jacobus Josephus, 5656 AE Eindhoven (NL)
(74) Representative: de Haan, Poul Erik

(57) **Abstract**

An anti-fouling system is used for protecting a surface against biofouling. Inductive power transfer is used to power an anti-fouling light source arrangement and a voltage multiplier is used at the receiver (secondary) side. The voltage multiplier enables a reduction in the optical impact of the secondary coils in the panel.

## Description

### FIELD OF THE INVENTION

The present disclosure relates to methods for preventing fouling, or commonly referred to as anti-fouling, of surfaces and to devices for performing these methods. The disclosure specifically relates to methods and devices for anti-fouling of the hull of ships.

### BACKGROUND OF THE INVENTION

Biofouling or biological fouling is the accumulation of microorganisms, plants, algae, and/or animals on surfaces. The variety among biofouling organisms is highly diverse and extends far beyond attachment of barnacles and seaweeds. According to some estimates, over 1700 species comprising over 4000 organisms are responsible for biofouling. Biofouling is divided into microfouling which includes biofilm formation and bacterial adhesion, and macrofouling which is the attachment of larger organisms. Due to the distinct chemistry and biology that determine what prevents organisms from settling, these organisms are also classified as hard or soft fouling types.

Calcareous (hard) fouling organisms include barnacles, encrusting bryozoans, mollusks, polychaete and other tube worms, and zebra mussels. Examples of non-calcareous (soft) fouling organisms are seaweed, hydroids, algae and biofilm "slime". Together, these organisms form a fouling community.

In several circumstances, biofouling creates substantial problems. Machinery stops working, water inlets get clogged, and hulls of ships suffer from increased drag. Hence the topic of anti-fouling, i.e. the process of removing or preventing fouling from forming, is well known.

In industrial processes, bio-dispersants can be used to control biofouling. In less controlled environments, organisms are killed or repelled with coatings using biocides, thermal treatments or pulses of energy. Nontoxic mechanical strategies that prevent organisms from attaching include choosing a material or coating with a slippery surface, or creation of nanoscale surface topologies similar to the skin of sharks and dolphins which only offer poor anchor points.

By way of example, biofouling on the hull of ships causes a severe increase in drag, and thus increased fuel consumption. It is estimated that an increase of up to 40% in fuel consumption can be attributed to biofouling. As large oil tankers or container transport ships can consume up to €200,000 a day in fuel, substantial savings are possible with an effective method of anti-bio fouling.

WO 2014/188347 discloses a method and system for preventing biofouling in which all of a surface, or a significant amount of a surface, to be kept clean from fouling (e.g. the hull of a ship) is covered with a layer that emits germicidal light, in particular UV light. Thus, it is known to adopt an optical method, in particular using ultra-violet light (UV). It is well-known that most micro-organisms are killed, rendered inactive or unable to reproduce with sufficient UV light. This effect is mainly governed by the total dose of UV light. A typical dose to kill 90% of a certain micro-organism is 10 mW-hours per square meter.

Ultraviolet (UV) is that part of electromagnetic light bounded by the lower wavelength extreme of the visible spectrum and the X-ray radiation band. The spectral range of UV light is by definition between 100 and 400 nm and is invisible to human eyes. Using the CIE classification the UV spectrum is subdivided into three bands:
UVA (long-wave) from 315 to 400 nm
UVB (medium- wave) from 280 to 315 nm
UVC (short-wave) from 100 to 280 nm

Various light sources for generating UV are known, such as low-pressure mercury discharge lamps, medium pressure mercury discharge lamps and dielectric barrier discharge lamps.

A preferred option, for example as proposed in WO 2014/188347 is low cost, lower power UV LEDs. LEDs can generally be included in smaller packages and consume less power than other types of light sources. LEDs can be manufactured to emit (UV) light of various desired wavelengths and their operating parameters, most notably the output power, can be controlled to a high degree. A suitable germicidal dose can easily be achieved with existing UV LEDs.

The UV LEDs can be formed in a panel to be placed over the surface to be protected. To power the UV LEDs, it is possible to use inductive power transfer from a primary side transmitter to a secondary side receiver in the panel. However, the pickup coil required at the secondary side takes up space, and this large coil surface needs to be compensated by adding extra LEDs. Increasing the number of LED is a problem as there is a higher power demand, and hence a higher current demand (for a given voltage), necessitating wider conductive tracks.

The primary side current can be reduced by increasing the number of primary coils, but this requires an increase in the number of secondary side coils to maintain a given turns ratio.

Thus, there are compromises needed to meet optical and electrical requirements of an inductive power transfer approach.

### SUMMARY OF THE INVENTION

The invention is defined by the claims.

According to examples in accordance with an aspect of the invention, there is provided an anti-fouling system for protecting a surface against biofouling, comprising:
a panel for emitting anti-fouling light and for mounting over the surface, comprising alight source arrangement for generating anti-fouling light and an inductive power receiver comprising one or more secondary windings for alignment with one or more primary windings of an inductive power transmitter,
wherein the inductive power receiver comprises a voltage multiplier.

The system preferably further comprises the inductive power transmitter for mounting over the surface and comprising the one or more primary windings.

The invention thus provides a panel as well as an overall system using the panel.

The overall system creates a galvanically isolated transformer between the power source for the power transmitter and the panel. To reduce the optical impact of the secondary coil (formed by the secondary winding(s)) in the panel, a voltage multiplier is used. This means that fewer windings can be used in the secondary coil, and with a low surface area, and thereby the optical performance of the panel can be improved. This in turn means that fewer light sources (e.g. LEDs) in the light source arrangement may be needed, giving a lower operating current and hence the option for thinner conductive tracks.

The primary coil (formed by the primary winding(s)) is designed to achieve a desired rate of change of magnetic flux, and the combination of the secondary coil design and the voltage multiplier design achieves the desired lighting performance.

An increase in the number of primary windings can be used to reduce the current required to generate desired magnetic flux characteristics, and the use of the voltage multiplier avoids the need for a correspondingly increased number of secondary windings. By enabling the number of secondary windings to be kept low, the coil losses caused by track resistance are reduced.

The voltage multiplier for example comprises a diode-capacitor circuit. This can be implemented with small discrete components, which can thus be arranged to take up less space, and have less optical impact, than the secondary coil windings they replace.

The voltage multiplier is for example a voltage quadrupler. This has been found to provide an optimal balance between the desired reduction in the number of secondary windings and the resulting loss in electrical efficiency.

The inductive power transmitter is for example for mounting against the surface and the panel is for mounting over the inductive power transmitter. The galvanically isolated panel is thus the outer layer.

The inductive power transmitter may comprise an elongate power strip, and the panel comprises an edge region which overlaps the elongate power strip, the inductive power receiver being formed in the edge region. Thus, the coil or coils take up a relatively small area of the panel. A grid may be formed of the power strips (e.g. arranged vertically) and panels (e.g. arranged horizontally).

The inductive power transmitter for example comprises a ferrite sheet below the primary windings, hence between the surface to be protected and the windings. The system efficiency can thus be kept high, e.g. close to 50%. The ferrite sheet is between the surface, e.g. a ship's hull, and the inductive transformer primary windings, to prevent eddy currents through the conductive hull (or other conductive layer which defines the surface to be protected).

The inductive power transmitter for example comprises at most 10 primary windings, and the power source for the inductive transmitter has an RMS voltage of 50V or less and delivers an RMS current of 3A or less.

The inductive power receiver for example comprises 10 or fewer secondary windings.

The inductive power receiver for example comprises secondary windings formed on or in a printed circuit board. The PCB material is typically an absorber for the UV light generated, so it should be as thin as possible.

The panel for example has a thickness of less than 5mm, for example less than 4mm, for example less than 3mm. This thickness typically includes the printed circuit board and a protective coating.

For example, the panel comprises a silicone material such as in the form of a coating or embedding material. This material may perform an optical function, e.g. light guiding, as well as a protective function. It may be selected to have relative high transparency to the UV light generated.

The inductive power transmitter for example comprises a resonant circuit with a resonant frequency of 50kHz to 1MHz, for example 50kHz to 200kHz, for example 60kHz to 90kHz.

The light source arrangement for example comprises an array of UV-C LEDs with wavelength between 270nm and 280nm.

The system may comprise a plurality of inductive power transmitters and a plurality of panels. One inductive power transmitter may be associated with one or more panels.

The invention also provides a method of protecting a surface against biofouling, comprising:
transmitting power wirelessly using an inductive power transmitter mounted over the surface;
receiving the wireless transmitted power using an inductive power receiver of a panel mounted over the surface;
performing voltage multiplication at the inductive power receiver; and
driving a light source arrangement using the voltage multiplied received power.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows an anti-fouling system applied to a ship for protecting the surface of the ship in contact with water, i.e. the hull surface;
Figure 2 shows a cross section (in a horizontal plane) through the inductive power transmitters and panels;
Figure 3 shows the coil arrangements in more detail;
Figure 4 shows an example of the structure of the panel;
Figure 5 shows a panel viewed from the light emitting surface;
Figure 6 shows the panel with LEDs, the secondary winding and passive components which together define a voltage multiplier;
Figure 7 shows an electric circuit implemented by the system; and
Figure 8 shows one example of a voltage quadrupler circuit as the voltage multiplier.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an anti-fouling system used for protecting a surface against biofouling. Inductive power transfer is used to power a light source arrangement for generating anti-fouling light and a voltage multiplier is used at the receiver (secondary) side. The voltage multiplier enables a reduction in the optical impact of the windings of the secondary side coil in the panel.

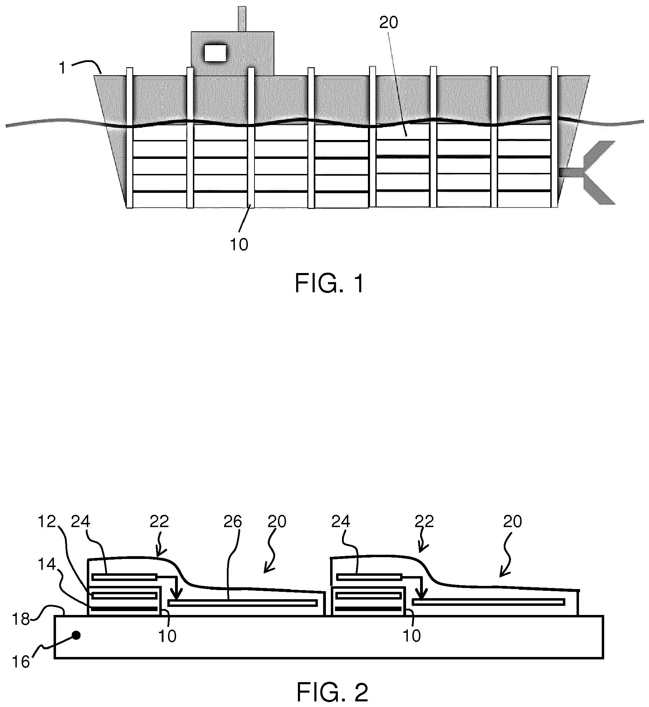

Figure 1 shows an anti-fouling system, which may be adapted in accordance with the invention, applied to a ship 1, for protecting the surface of the ship in contact with water, i.e. the hull surface.

The anti-fouling system comprises a set of an inductive power transmitters 10 mounted over the surface. They take the form of power feeding lines which extend vertically against the hull. At the upper ends, the feeding lines connect to a source of electric power (not shown). The inductive power transmitters each comprise a set of one or more primary windings. A set of primary windings (whether there is one or more than one winding) is referred to as a primary coil in this document.

A set of panels 20 is also mounted over the surface. The panels each comprise a light source arrangement and an inductive power receiver with a set of one or more secondary windings for alignment with the set of primary windings. A set of secondary windings (whether there is one or more than one winding) is referred to as a secondary coil in this document. Thus, the term "coil" generally is used to denote a set of windings forming one side of a transformer.

As will be clear from the description below, there may be multiple coils on each power feeding line, for example one or more coils along the power feeding line at the location of each panel.

As explained in more detail below, the inductive power receiver comprises a voltage multiplier.

The invention provides the overall system, the panels in isolation, and a lighting method.

Figure 2 shows a cross section (in a horizontal plane) through the power feeding lines (the inductive power transmitters 10) and through the panels 20. The inductive power transmitters comprise a primary coil 12 and a ferrite sheet 14 between the windings of the primary coil and the metal of the hull 16 of the ship. The surface 18 of the hull is the surface to be protected from fouling. The ferrite sheets prevent Eddy currents in the metal of the ship's hull 16 thereby increasing the efficiency of energy transfer.

In the example shown, the surface 18 is essentially fully covered by the panels. Thus, the surface 18 is protected by the panels, and it is the exposed surface of the panels which is vulnerable to fouling. Thus, the lighting provided by the panels aims to prevent the formation of fouling organisms on the surface of the panels.

However, this is still to be understood as forming the system for protecting the hull surface against biofouling (in that without the system, the hull surface will suffer from biofouling).

An alternative arrangement for example may have panels which only cover a small fraction of the surface to be protected, and the light is directed or guided towards the surface to be protected. In such a case, a major part of the hull surface is indeed exposed to water and therefor susceptible to biofouling.

In the example shown in Figure 2, the inductive power transmitters 10 are mounted against the hull surface 18 and the panels 20 are mounted over the inductive power transmitters.

In particular, an edge region 22 of each panel 20 overlaps the feeding lines. The panels 20 each have a secondary coil 24 located in this edge region, and a light source arrangement 26.

The secondary windings are aligned with the primary windings to provide inductive power transfer. The wirelessly transmitted power is used by the panels 20 to power the light source arrangements 26.

The primary coils may be formed on or within a printed circuit board of the feeding lines, and the secondary coils may also be formed on or within a printed circuit board of the panel. The light source arrangement may also be formed on a printed circuit board, which may be separate to, or the same as, the printed circuit board of the secondary coils. A shared flexible printed circuit board may for example allow the panels to adapt to the contour of the underlying feeding lines. Instead, there may be separate printed circuit boards in the panel and an electrical connection between them. Alternatively, the light source arrangement may be formed as a wire grid structure. This reduces the PCB area since a PCB is needed only for the secondary coils.

The printed circuit boards are not shown in the figures, to keep the shown structure simple.

The primary coils of the inductive power transmitters 10 may for example be supplied with a 100 kHz to 150 kHz sinewave during operation of the light emitting system. To compensate for a capacitive leakage current to the hull 16 at the position of the feeding lines, the feeding lines may further be provided with a capacitor to implement a low pass filter . This is for example of interest if high efficiency switched amplifiers are used to generate the AC supply. In such a case, the low pass filter is used to residual higher frequency harmonics of the amplifiers.

An alternative is to use a resonant circuit to generate the AC supply. For example, each feeding line may comprise a resonant circuit, based on a capacitive resonant circuit, with a resonance in the range 60kHz to 90kHz.

Generally, the frequency of operation (resonant or driven) may be in the range 50kHz to 1MHz, for example 50kHz to 200kHz, for example 60kHz to 90kHz.

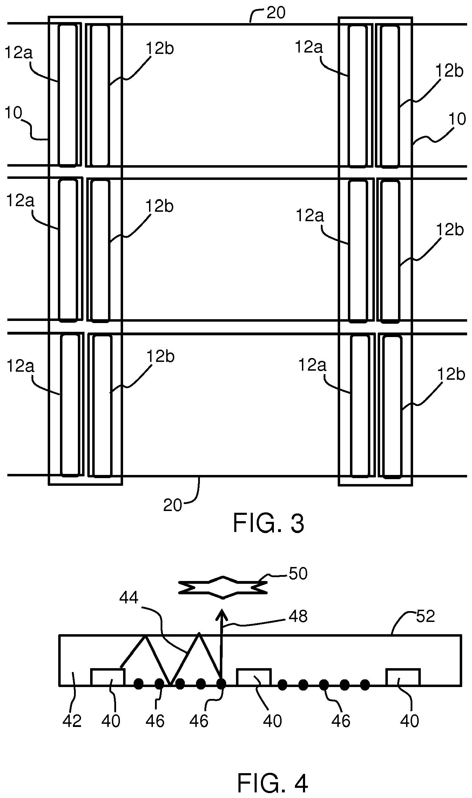

Figure 3 shows the coil arrangements.

The example of Figure 2 has the panels overlapping an associated feeding line at one edge. In Figure 4, the panels 20 overlap feeding lines 10 at both lateral edges, and each feeding line 10 has pairs of primary coils arranged along its length. One coil of a pair is for powering a panel to one side and the coil of the pair is for powering a panel to the other side. In this way, each panel is supplied by power from both sides. This reduces the length of power lines needed to the light source arrangement.

All coils of the feeding lines can have the same phase, which contributes to electric redundancy of the light emitting system 40. The light source arrangements 26 can still function in their entirety if a feeding line is broken. In that respect, the feeding lines may be designed to deliver electric power at an increased level of two times a normal level.

Thus, there may be one coil assembly (i.e. primary coil and secondary coil) per panel (Figure 2) or two coil assemblies per panel (Figure 3).

There may for example be between 2 and 50 panels per feeding line, for example 20 rows of individual tiles connected to a feeding line.

In the example shown, the feeding lines extend in a substantially vertical orientation along the side of the ship. However, any suitable arrangement of feeding lines is possible. The feeding lines may for example cover welding seams and/or other surface irregularities of the ship's hull.

Figure 4 shows an example of the structure of the panel 20, having a plurality of light sources 40 which in this example are side-emitting UV-C LEDs, wherein the light is emitted primarily from a side of the LED, and more or less parallel to the surface. The light sources 40 are encapsulated in a liquid-tight optical medium 42 to guide at least part of the light 44 emitted from the light sources 40 via total internal reflection through the optical medium.

Optical structures 46 are provided to disrupt the total internal reflection and scatter light, and then guide the scattered light 48 out of the optical medium 42 towards an target for the light, which is an area where a biofouling organism is present.

A biofouling organism on the surface 52 will directly receive the light scattered light 48 before it enters the water.

Furthermore, some of the internally scattered light 48 that does enter the water will encounter external scattering sites. This creates illumination 50 within the water, some of which will also reflect back to the surface 52 of the panel 20 where biofouling is to be prevented.

The illumination means that single cell bio-mechanisms at the surface 52 will stop growing and dividing, and will therefor die under influence of the UV-C light.

The optical medium is relatively thin so that the panel may be considered to be a two-dimensional structure. The optical structures 46 to scatter light may be spread in one or more portions of the optical medium material, possibly throughout all of it, and the light output may be generally homogeneous or else localized.

Internal scattering centers with different structural properties may be combined to provide optical and well as structural characteristics, such as resistance to wear and/or impact. Suitable scatterers comprise opaque objects but largely translucent objects maybe used as well, e.g. small air bubbles, glass and/or silica; a requirement is merely that a change in refractive index occurs for the wavelength(s) used.

The principle of light guiding and spreading light over a surface is well-known and widely applied in various fields. Here, the principle is applied to UV light for the purpose of anti-fouling.

To maintain the conditions for total internal reflection, the index of refraction of the light guiding material should be higher than that of the surrounding medium. However, the use of (partly) reflecting coatings on the light guide and/or the use of the reflective properties of the protected surface, e.g. the hull of a ship, itself can also be used to establish the conditions for guiding the light through the optical medium.

In the example above, the panels form a new surface over the surface to be protected, and light is directed outwardly from the surface to be protected. However, an alternative is for the panel to be spaced over the surface to be protected and to direct light back towards the surface to be protected.

A small air gap may then be introduced between the light source arrangement of the panel and the surface to be protected. UV light may travel better, with less absorption, in air than in an optical medium, even when this optical medium is designed as a light guiding material.

As most materials have a (very) limited transmittance for UV light, care has to be taken in the design of the optical medium. As a result, a relatively fine pitch of low power LEDs can be chosen, to minimize the distance light has to travel through the optical medium.

In one example, the optical medium 42 comprises a silicone, and one which is designed to have good UV-C transparency.

A solid encapsulation may be used, as shown in Figure 4. However, a hollow structure may instead be used, such as a silicone mat with spacers that keep it a small distance away from the protected surface. This creates air channels, through which the UV light can propagate with higher efficiency. Use of gas filled channels provided by such structures allows distributing the UV light over significant distances in a optical medium of material that would otherwise absorb the UV light too strongly to be useful for anti-fouling. Similarly, separate pockets may be formed.

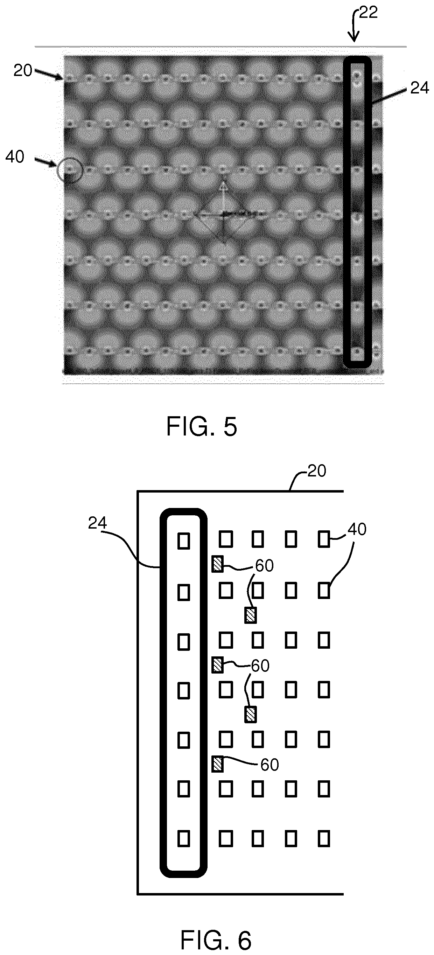

Figure 5 shows a panel 20 viewed from the front, and showing the illumination pattern. The panel comprises a two dimensional array of the LEDs 40 and the primary coil 24 at an edge region 22.

In accordance with the invention, the inductive power receiver comprises a voltage multiplier. Optionally, the voltage multiplier implements rectification as well.

The voltage multiplier can be formed of passive components, such as diodes and capacitors forming a diode-capacitor circuit. The LEDs 40 are provided on a printed circuit board, and the printed circuit board traces also define the windings of the secondary coil. The components of the voltage multiplier may also be provided on the same substrate, interspersed with the LEDs.

Figure 6 shows the panel with LEDs 40, the secondary coil 24 and passive components 60 (e.g. capacitors and diodes) which together define the voltage multiplier. The voltage multiplier is for example a voltage quadrupler. There are PCB tracks for configuring the circuit of the voltage multiplier from the discrete components.

A voltage multiplier may be distributed over an area as shown in Figure 6, or it may be provided at a single location. There may be multiple voltage multipliers spread over the PCB. For large lighting tiles, the voltage multipliers may be distributed over the tiles, and be electrically connected to deliver power to the light sources at a local region of the tile.

The use of inductive power transfer creates a galvanically isolated transformer between the power source and the panel. The voltage multiplier means that fewer secondary coil windings can be used, and thereby the optical performance of the panel can be improved. Figure 6 shows that LEDs may be provided within the inner area of the windings, in order to maintain a uniform distribution of LEDs over the panel. The improved optical performance means that fewer light sources (e.g. LEDs) in the light source arrangement may be needed, giving a lower operating current and hence the option for thinner conductive tracks.

The primary coils (size, materials, and number) are designed to achieve a desired rate of change of magnetic flux, and the combination of the secondary coil design and the voltage multiplier design achieves the desired lighting performance.

An increase in the number of primary windings can be used to reduce the current required to generate a desired magnetic flux, and the use of the voltage multiplier avoids the need for a correspondingly increased number of secondary windings. By enabling the number of secondary windings to be kept low, the coil losses caused by track resistance are reduced.

By way of example each coil in the power transmitter may have 10 or less windings.

For example, a primary coil with 1 primary side winding may be driven with a relatively large current e.g. 2A rms requiring a relatively low voltage e.g. 5V rms. A coil with 5 primary side windings may be driven with a reduced current e.g. 1A rms requiring a greater voltage e.g. 10V rms. A coil with 10 primary side windings may be driven with a relatively small current e.g. 0.4A rms requiring a relatively high voltage (e.g. 24V rms).

Without the voltage multiplication, the coils in the secondary side may for example require 20 windings to create a desired 48V supply from a coil with 10 primary side windings at 24V rms.

If a voltage doubler is used, the coils in the secondary side may then require 10 windings. If a voltage quadrupler is used, the coils in the secondary side may then require 5 windings.

A typical secondary side current is 0.1A and a typical desired secondary side voltage is around 40V. For safety, a maximum voltage of 50V rms (by way of example only) may be considered. The system is designed or operate below the maximum voltage, taking into accout all the characteristics of the inductive coupling and the spreading of currents. For a given operating voltage, the required current depends on the required power. A higher voltage enables a lower current and vice versa.

The feeding lines for example make use of a PCB with thickness of less than 1mm, for example 0.5mm, created a molded structure thickness of around 3mm.

The panels for example have a PCB thickness of 0.8mm, and the total thickness with the silicone of below 5mm, for example in the range 2mm to 4mm.

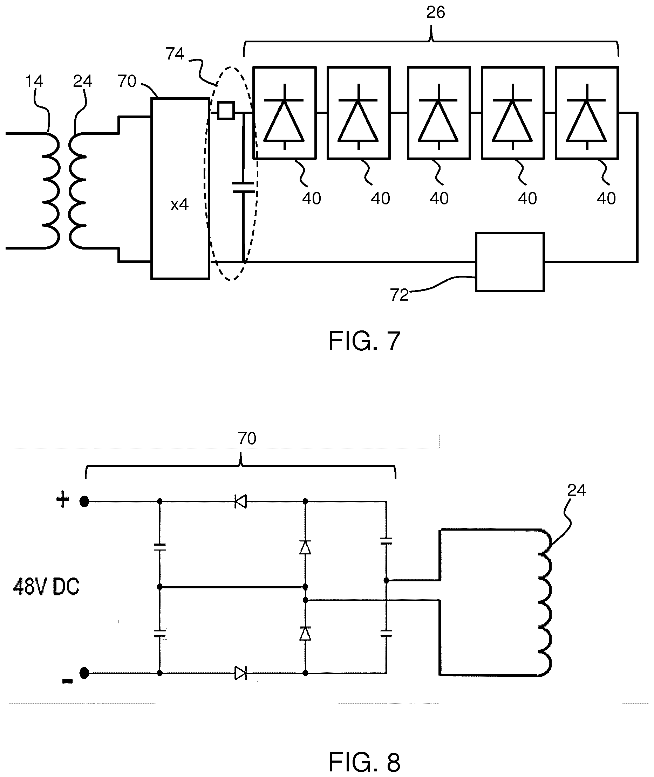

Figure 7 shows an electric circuit implemented by the system.

It shows the set 14 of primary windings, the set 24 of secondary windings, the voltage multiplier 70, and the series connection of UV-C LEDs. The LEDs are driven by a programmable current source 72. A low pass filter 74 is formed by a resistor and capacitor.

Figure 8 shows one example of a voltage quadrupler circuit as the voltage multiplier 70. The circuit is a known diode-capacitor circuit formed using discrete components which are mounted on the PCB of the panel as explained with reference to Figure 6. The circuit functions as both a rectifier and a voltage multiplier, and thereby generates a DC output voltage from an AC input from the receiver coil.

The circuit has four capacitors. The input voltage charges each capacitor, and the diode connections between the capacitors provide successive addition of the voltages.

There are various different voltage multiplier circuit topologies based on diodes and capacitors, and this is just one example.

The invention is of particular interest for marine objects although not limited to objects for use in seawater, but also in any type of water that is known to contain biofouling organisms. Examples of marine objects include ships and other vessels, marine stations, sea-based oil or gas installations, buoyancy devices, support structures for wind turbines at sea, structures for harvesting wave/tidal energy, sea chests, underwater tools, etc.

In preferred examples, the anit-fouling light sources are UV LEDs as explained above. A grid of UV LEDs may be encapsulated in a liquid-tight encapsulation, of which silicone is only one example. The UV LEDs may be electrically connected in a series and/or parallel arrangement. The UV LEDs are for example packaged surface mount LEDs, in which case they already may include an optical element to distribute the light emitted from the LED package across a wide emission angle. In other embodiments, the UV LEDs may be LED dies, typically not comprising optical elements but being significantly thinner than packaged LEDs. As an example, LED dies could be picked and placed onto a surface of the optical medium

The silicone material can be selected to provide optical transmission for UV light with little loss compared to other materials. This is in particular the case for shorter wavelength light, e.g. UV light with wavelengths below 300 nm. A particularly efficient group of silicone materials is, or at least comprises, so-called methyl silicones, according to the general chemical formula CH₃[Si(CH₃)₂O]ₙSi(CH₃)₃, with "n" indicating any suitable integral.

Silicone materials are also flexible and resilient so that they are robust, durable and capable of withstanding compression such as due to bumps, collisions etc. of objects against the surface, e.g. bumping of a ship against a quay. Furthermore, deformation due to temperature fluctuation, pounding by waves, flexion of the ship over swell etc. may be accommodated.

At least part of light emitted by the one or more light sources may be spread in a direction having a component substantially parallel to the surface to be protected. This facilitates distributing the light over significant distances along the protected surface, or the application surface of the foil, which assists in obtaining a suitable intensity distribution of the anti-fouling light.

A wavelength conversion material may be comprised in the optical medium and at least part of the anti-fouling light may be generated by photo-exciting the wavelength conversion material with light having a first wavelength causing the wavelength conversion material to emit the anti-fouling light at another wavelength. The wavelength conversion material may be provided as an up-conversion phosphor, quantum dots, nonlinear media such as one or more photonic crystal fibers etc. Since absorption and/or scattering losses in the optical medium for light of different, mostly longer, wavelengths than UV light tend to be less pronounced in the optical media, it may be more energy-efficient to generate non-UV light and transmit that through the optical medium and to generate UV anti-fouling light at or near the desired location of use thereof (i.e. emission form the surface into the liquid environment).

The example shown makes use of side-emitting LEDs and optical scattering sites. However, light spreading arrangements may be used to create the sideways light. For example, a cone may be arranged in the optical medium and positioned opposite the light source, where the opposing cone has a surface area with a 45° angle perpendicular to the protected surface for reflecting light emitted by the light source perpendicular to said surface in a direction substantially parallel to said surface.

The LEDs are preferably DC driven. However, a pair of back to back parallel LEDs may be driven by an AC drive signal.

As mentioned above the LEDs are preferably mounted on a PCB, and PCB tracks (on the PCB surface or internally within layers of the PCB) form the receiver coil. However, the LED grid may instead be formed by connecting LEDs to a connection nodes of a freestanding wire structure by soldering, gluing or any other known electrical connection technique. This may be combined with a secondary coil on a smaller PCB.

The invention can be applied to a wide variety of fields. Almost any object coming into contact with natural water, will over time be subject to biofouling. This can hinder e.g. water inlets of desalination plants, block pipes of pumping stations, or even cover the walls and bottom of an outdoor pool. All of these applications would benefit from the presently provided method, lighting modules and/or system, i.e. an effective thin additional surface layer, which prevents biofouling on the entire surface area.

Although UV light is the preferred solution, other wavelengths are envisaged as well. Non-UV light (visible light) is also effective against biofouling. Typical micro- organisms are less sensitive to non-UV light than to UV light, but a much higher dose can be generated in the visible spectrum per unit input power to the light sources.

UV LEDs are an ideal source for thin light emitting surfaces. However, UV sources other than LEDs can also be used, such as low pressure mercury vapor lamps. The form factor of these light sources are quite different; mainly the source is much bigger. This results in different optical designs, to distribute all the light from a single source over a large area. Further, a significant contribution of light in desired wavelengths and/or wavelength combinations may be produced. Instead of using a thin layer that emits UV light outward in a direction away from the protected surface in order to avoid bio-fouling, biofouling could potentially also be removed by applying UV light from the outside in the direction of the protected surface, as explained above. The panel may instead emit anti-fouling light both in directions towards and away from the surface to be protected.

In the examples above, the panel overlaps the feeding lines. This provides galvanic isolation between the power supply and the structure which is exposed to the water. Instead, the feeding lines may be provided over the panels. A separate electrical isolation may be provided (e.g. at the top of the feeding lines). The surface of the feeding lines will then be susceptible to biofouling, so it should then be ensured that light reaches the surface of the feeding lines, either by transmission through the feeding lines or by reflection or waveguide transmission within the panels. Thus, the inductive power transmitter and the panel are both for mounting over the surface, but in either order.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. If the term "adapted to" is used in the claims or description, it is noted the term "adapted to" is intended to be equivalent to the term "configured to". Any reference signs in the claims should not be construed as limiting the scope.

## Claims

1. An anti-fouling system for protecting a surface (16) against biofouling, comprising:
a panel (20) for emitting anti-fouling light the panel for mounting over the surface, comprising an anti-fouling light source arrangement for generating the anti-fouling light and an inductive power receiver (24) comprising one or more secondary windings for alignment with one or more primary windings of an inductive power transmitter,
wherein the inductive power receiver (24) comprises a voltage multiplier (70).

2. An anti-fouling system as claimed in claim 1, wherein the voltage multiplier (70) comprises a diode-capacitor circuit.

3. An anti-fouling system as claimed in claim 1 or 2, wherein the voltage multiplier (70) is a voltage quadrupler.

4. An anti-fouling system as claimed in any one of claims 1 to 3, wherein the inductive power receiver (24) comprises 10 or fewer secondary windings formed on or in a printed circuit board.

5. An anti-fouling system as claimed in any one of claims 1 to 4, wherein the panel (20) has a thickness of less than 5mm, for example less than 4mm, for example less than 3mm.

6. An anti-fouling system as claimed in any one of claims 1 to 5, wherein the panel comprises a printed circuit board at least for the one or more secondary windings and a silicone coating.

7. An anti-fouling system as claimed in any one of claims 1 to 6, wherein the anti-fouling light source arrangement comprises one or more UV-C LEDs for emitting the antifouling light with wavelength between 270nm and 280nm.

8. An anti-fouling system as claimed in any one of claims 1 to 7, further comprising the inductive power transmitter (12) for mounting over the surface (16) and comprising the one or more primary windings.

9. An anti-fouling system as claimed in claim 8, wherein the inductive power transmitter is for mounting against the surface (16) and the panel (20) is for mounting over the inductive power transmitter.

10. An anti-fouling system as claimed in claim 8 or 9, wherein the inductive power transmitter (10) comprises an elongate power strip, and the panel comprises an edge region (22) which overlaps the elongate power strip (10), the inductive power receiver (24) being formed in the edge region (22).

11. An anti-fouling system as claimed in any one of claims 8 to 10, wherein inductive power transmitter comprises a ferrite sheet (14) below the windings.

12. An anti-fouling system as claimed in any one of claims 8 to 11, wherein the inductive power transmitter (12) comprises at most 10 primary windings, and the power source for the inductive transmitter has an RMS voltage of 50V or less and delivers an RMS current of 3A or less.

13. An anti-fouling system as claimed in any one of claims 8 to 12, wherein the inductive power transmitter (12) comprises a resonant circuit with a resonant frequency of 50kHz to 1MHz, for example 50kHz to 200kHz, for example 60kHz to 90kHz.

14. An anti-fouling system as claimed in any one of claims 8 to 13, comprising a plurality of inductive power transmitters (10) and a plurality of panels (20).

15. A method of protecting a surface (16) against biofouling, comprising:
transmitting power wirelessly using an inductive power transmitter (12) mounted over the surface;
receiving the wireless transmitted power using an inductive power receiver (24) of a panel (20) for providing antifouling light and mounted over the surface;
performing voltage multiplication at the inductive power receiver (24); and
driving an anti-fouling light source arrangement (26) using the voltage multiplied received power.
